(19) Europäisches Patentamt
European Patent Office
Office européen des brevets

(11) Veröffentlichungsnummer : **0 340 708 B1**

# (12) EUROPÄISCHE PATENTSCHRIFT

(45) Veröffentlichungstag der Patentschrift : **12.08.92 Patentblatt 92/33**

(51) Int. Cl.$^5$ : **C07D 209/48**, C07D 317/28, C07D 319/06

(21) Anmeldenummer : **89107884.2**

(22) Anmeldetag : **29.04.89**

## (54) Verfahren zur Herstellung von 3-(3,4,5,6-Tetrahydrophthalimido)-benzaldehyden.

Die Akte enthält technische Angaben, die nach dem Eingang der Anmeldung eingereicht wurden und die nicht in dieserPatentschrift enthalten sind.

(30) Priorität : **04.05.88 DE 3815042**

(43) Veröffentlichungstag der Anmeldung : **08.11.89 Patentblatt 89/45**

(45) Bekanntmachung des Hinweises auf die Patenterteilung : **12.08.92 Patentblatt 92/33**

(84) Benannte Vertragsstaaten : **BE CH DE FR GB IT LI NL**

(56) Entgegenhaltungen :
**EP-A- 0 061 741**
**DE-A- 3 819 464**

(56) Entgegenhaltungen :
**CHEMICAL ABSTRACTS, Band 103, 1985, Seite 881, Zusammenfassung Nr. 215163m, Columbus, Ohio, US; & JP-A-60 152 465(MITSUBISHI CHEMICAL INDUSTRIES CO., LTD) 10-08-1985**
**CHEMICAL ABSTRACTS, Band 73, 1970, Seite 371, Zusammenfassung Nr. 109769r, Columbus, Ohio, US; D. St. CLAIR BLACK et al.:"Metal template reactions. III. Synthesis of macrocyclic quadridentate nickel(II) complexes from a suitable diaminodialdehyde",&& AUST. J. CHEM. 1970, 23(10), 2039-2053**
**J.March, Advanced Organic Chemistry, McGraw-Hill, Tokyo, p.490,491.**

(73) Patentinhaber : **BASF Aktiengesellschaft**
**Carl-Bosch-Strasse 38**
**W-6700 Ludwigshafen (DE)**

(72) Erfinder : **Fisher, Klaus, Dr.**
**Gabelsbergerstrasse 7**
**W-6720 Speyer (DE)**
Erfinder : **Rueb, Lothar, Dr.**
**Zum Weidentor 4**
**W-6720 Speyer (DE)**
Erfinder : **Plath, Peter, Dr.**
**Hans-Balcke-Strasse 13**
**W-6710 Frankenthal (DE)**

EP 0 340 708 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues Verfahren zur Herstellung von 3-(3,4,5,6-Tetrahydrophthalimido)-benzaldehyden (I).

Weiterhin betrifft die Erfindung neue 3-(3,4,5,6-Tetrahydrophthalimido)-benzaldehyde der allgemeinen Formel Ia

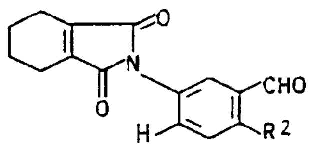

Ia,

in der $R^2$ für Halogen steht, sowie neue Vorstufen der allgemeinen Formel IIa

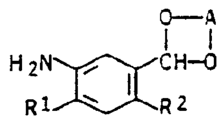

IIa,

in der $R^1$ Wasserstoff oder Halogen, $R^2$ Halogen und A eine 1,2-Ethylen- oder 1,3-Propylenbrücke, die beide ihrerseits bis zu drei $C_1$-$C_3$-Alkylgruppen tragen können, bedeuten.

Gemäß der GB-A 2,150,929 sind Tetrahydrophthalimide, die am Stickstoffatom des Tetrahydrophthalimid-Grundkörpers einen Phenylring tragen, der u.a. durch eine Aldehyd- oder Ketogruppe substituiert sein kann, herbizid wirksam. Sie können durch Friedel-Crafts-Acylierung von N-Phenyl-tetrahydrophthalimiden erhalten werden.

In der DE-A 38 19 464 werden herbizid wirksame N-(Heterocyclylidenmethyl)-pent-yl-3,4,5,6-tetrahydrophthalimide sowie deren Herstellung, beispielsweise aus Triarylphosphoranen und N-(3-Formylphenyl)-tetrahydrophthalimiden, beschrieben. Die N-(3-Formylphenyl)-tetrahydrophthalimid-Zwischenprodukte lassen sich gemäß dieser Druckschrift durch katalytische Hydrierung von Nitrobenzaldehydacetalen an Edelmetallkatalysatoren oder Katalysatoren wie Raney-Nickel und Kondensation der so erhaltenen Aminobenzaldehydderivate mit Tetrahydrophthalsäureanhydrid herstellen.

Desweiteren ist aus der JP-A 60/152 465 bekannt, 3-Nitrobenzaldehyde mit Eisen in essigsaurem Medium zu den entsprechenden 3-Aminobenzaldehyden zu reduzieren und diese anschließend mit 3,4,5,6-Tetrahydrophthalsäureanhydrid in die Verbindungen des Typs I zu überführen:

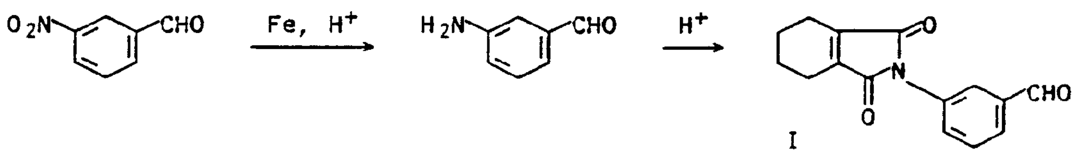


I

Nachteilig an diesem Verfahren ist, daß die Reduktion mit Eisen verfahrenstechnisch umständlich ist und daß die Aminobenzaldehyde zu Selbstkondensation neigen, weswegen man nur unbefriedigende Ausbeuten an den Verfahrensprodukten erzielt, die darüber hinaus nur in ungenügender Reinheit anfallen.

Der Erfindung lag allgemein ein universell anwendbares Verfahren zur Herstellung der 3-(3,4,5,6-Tetrahydrophthalimido)-benzaldehyde des Typs I als Aufgabe zugrunde, wobei sich die Aufgabe speziell auf die neuen Verbindungen Ia und die neuen Vorstufen IIa erstreckte.

Demgemäß wurde ein neues Verfahren zur Herstellung von 3-(3,4,5,6-Tetrahydrophthalimido)-benzaldehyden des Typs I gefunden, welches dadurch gekennzeichnet ist, daß man

a) die Aldehydgruppe eines 3-Nitrobenzaldehyds III in einem inerten organischen Lösungsmittel in Gegenwart eines sauren Katalysators mit einem Alkandiol HO-A-OH IV, worin A für eine 1,2-Ethylen- oder 1,3-Propylenkette steht, wobei diese Ketten ihrerseits bis zu drei $C_1$-$C_3$-Alkylgruppen tragen können, zum entsprechenden cyclischen Acetal V umsetzt,

b) das cyclische Acetal V mit Wasserstoff katalytisch zur entsprechenden Aminoverbindung II reduziert und

c) dieses Aminophenylacetal II in einem sauren Reaktionsmedium mit 3,4,5,6-Tetrahydrophthalsäureanhydrid kondensiert, wobei das bei der Cyclisierung freigesetzte Wasser die Entacetalisierung der Formylgruppe bewirkt und direkt der gewünschte (3,4,5,6-Tetrahydrophthalimido)-benzaldehyd I entsteht.

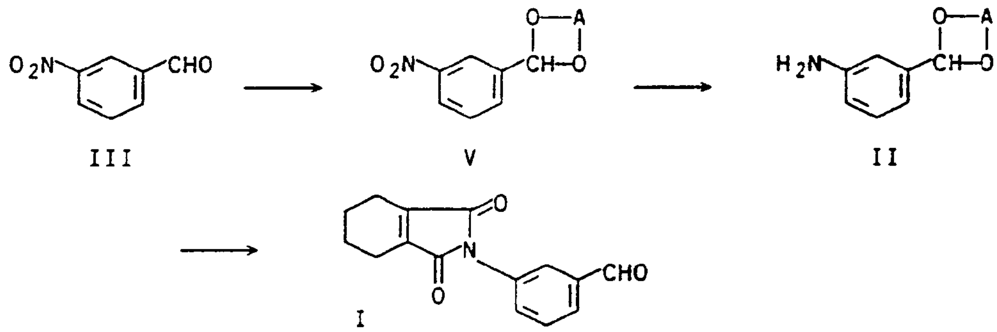

III V II

I

Weiterhin wurden die eingangs definierten neuen Verbindungen Ia und IIa gefunden

Die Umsetzung des Nitrophenylaldehyds III zum Nitrophenylacetal V erfolgt in an sich bekannter Weise [Houben-Weyl Bd. VI.3, S. 203ff] in einem inerten organischen Lösungsmittel in Gegenwart eines sauren Katalysators bei Temperaturen von 25 bis 150°C, vorzugsweise 80 bis 120°C, wobei das während der Reaktion entstehende Wasser kontinuierlich dem Reaktionsgemisch entzogen wird.

Geeignete saure Katalysatoren sind beispielsweise anorganische Säuren wie Salzsäure, Schwefelsäure und Phosphorsäure; organische Carbon-, Sulfon-und Phosphonsäuren wie insbesondere aliphatische und aromatische Sulfonsäuren (Methansulfonsäure, Trifluormethansulfonsäure, Benzolsulfonsäure und Toluolsulfonsäure) und Salze wie Eisen-III-chlorid, Zink-II-chlorid und Natriumhydrogensulfat, wobei die Katalysatorkonzentration 0,01 bis 10 Mol.%, vorzugsweise 0,1 bis 1 Mol.%, bezogen auf das Edukt III beträgt. Als Lösungsmittel werden bevorzugt inerte organische Lösungsmittel, die in der Lage sind mit Wasser Aceotrope zu bilden, sofern sich die Edukte und/oder die Produkte darin zumindest teilweise lösen. Geeignet sind beispielsweise aromatische Kohlenwasserstoffe wie Benzol, Toluol und Xylol; aliphatische Kohlenwasserstoffe wie Hexan, Heptan und Cyclohexan; chlorierte Kohlenwasserstoffe wie Chloroform, Tetrachlorkohlenstoff, 1,2-Dichlorethan und Chlorbenzol, sowie Ether wie Diethylether, Methyl-butylether und Diisopropylether.

Die Isolierung des Nitrophenylacetals V erfolgt nach üblichen Methoden.

Das so erhaltene Acetal V wird anschließend mit Wasserstoff an einem Metall- oder Edelmetallkatalysator zum Aminophenylacetal II reduziert.

Als Katalysatoren kommen hierbei Platin, Palladium, Rhodium, Ruthenium und Rhenium in Betracht, wobei Raney-Nickel besonders vorteilhaft ist. Die Katalysatorkonzentration liegt vorteilhaft bei 10 bis 30 Gew.%, bezogen auf das Edukt V. Die Umsetzung wird in einem inerten protischen oder aprotisch polaren organischen Lösungsmittel wie Alkohole wie Methanol, Ethanol, Iso-Propanol und Glykol, Carbonsäuren wie Essigsäure und Propionsäure oder Ethern wie vorstehend genannt sowie Tetrahydrofuran und Dioxan oder entsprechenden Gemischen bei Temperaturen von 0 bis 100°C, vorzugsweise 25 bis 50°C und Wasserstoffdrucken von 1 bis 50 bar, insbesondere 1 bis 10 bar durchgeführt.

Das Aminophenylacetal II wird aus dem Reaktionsgemisch in allgemein üblicher Weise isoliert und anschließend nach an sich bekannten Methoden mit 3,4,5,6-Tetrahydrophthalsäureanhydrid kondensiert.

Die Kondensation wird üblicherweise in protisch polaren Lösungsmitteln wie Essigsäure und Propionsäure oder entsprechenden Gemischen bei Temperaturen von 50 bis 150°C, vorzugsweise 70 bis 120°C durchgeführt. Unter diesen Reaktionsbedingungen tritt gleichzeitig eine Spaltung der Acetalgruppe zur Aldehydfunktion auf.

Die für das Verfahren benötigten Nitrobenzaldehyde III sind aus der Literatur bekannt oder können nach bekannten Verfahren (Houben-Weyl, Bd. E3) hergestellt werden.

Als Ausgangsverbindungen für das erfindungsgemäße Verfahren eignen sich neben dem 3-Nitrobenzaldehyd als Stammkörper auch kernsubstituierte Derivate, sofern deren Substituenten sich unter den Reaktionsbedingungen inert verhalten. Als Substituenten kommen beispielsweise Halogen wie insbesondere Fluor, Chlor und Brom, die Hydroxylgruppe, Alkylgruppen, Alkoxygruppen, Dialkylaminogruppen und Alkoxycarbonylgruppen in Betracht, wobei Alkylreste in diesen Gruppen vorzugsweise bis zu 12 C-Atome enthalten und ihrerseits Halogenatome wie vorzugsweise Fluor und Chlor, Hydroxyl- und $C_1$-$C_4$-Alkoxygruppen tragen können.

Bevorzugt werden diejenigen Verbindungen III und damit diejenigen Verfahrensprodukte I welche den neuen Verbindungen Ia bzw. IIa entsprechen, darunter vor allem die in den nachfolgenden Tabellen A und B genannten Substanzen.

Tabelle A

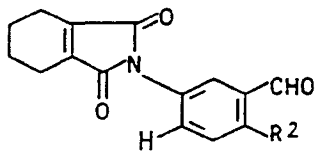

Ia

| $R^1$ | $R^2$ |
|---|---|
| H | F |
| H | Cl |
| H | Br |
| F | F |
| F | Br |
| Cl | F |
| Cl | Cl |
| Cl | Br |
| Br | F |
| Br | Cl |
| Br | Br |

Tabelle B

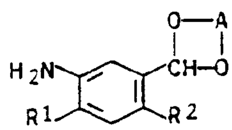

IIa

| $R^1$ | $R^2$ | A |
|---|---|---|
| H | F | $-CH_2CH_2-$ |
| H | Cl | $-CH_2CH_2-$ |
| H | Br | $-CH_2CH_2-$ |
| F | F | $-CH_2CH_2-$ |
| F | Br | $-CH_2CH_2-$ |
| Cl | F | $-CH_2CH_2-$ |
| Cl | Cl | $-CH_2CH_2-$ |
| Cl | Br | $-CH_2CH_2-$ |
| Br | F | $-CH_2CH_2-$ |
| Br | Cl | $-CH_2CH_2-$ |
| Br | Br | $-CH_2CH_2-$ |
| H | F | $-CH_2CH_2CH_2-$ |
| H | Cl | $-CH_2CH_2CH_2-$ |
| H | Br | $-CH_2CH_2CH_2-$ |
| F | F | $-CH_2CH_2CH_2-$ |
| F | Br | $-CH_2CH_2CH_2-$ |
| Cl | F | $-CH_2CH_2CH_2-$ |
| Cl | Cl | $-CH_2CH_2CH_2-$ |
| Cl | Br | $-CH_2CH_2CH_2-$ |
| Br | F | $-CH_2CH_2CH_2-$ |
| Br | Cl | $-CH_2CH_2CH_2-$ |
| Br | Br | $-CH_2CH_2CH_2-$ |

Die 3-(3,4,5,6-Tetrahydrophthalimido)-benzaldehyde I sind wertvolle Zwischenprodukte zur Herstellung von Herbiziden und Wachstumsregulatoren der allgemeinen Struktur VI

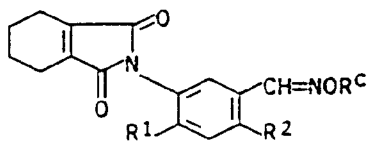

VI

Derartige Wirkstoffe sind beispielsweise in DE-A 3 607 300 und JP 60/152 465 beschrieben.

Das neue Verfahren zur Herstellung der Verbindungen I ist nachstehend anhand von Beispielen erläutert:

Beispiel 1

Herstellung von 2-Chlor-5-(3,4,5,6-tetrahydrophthalimido)-benzaldehyd

a) Eine Lösung aus 371 g (2,00 mol) 2-Chlor-5-nitrobenzaldehyd, 137 g (2,2 mol) Ethylenglykol, 1 g p-Toluolsulfonsäure und 1,5 l Toluol wurde 5 Stunden bei Siedetemperatur gerührt, wobei das Reaktionswasser kontinuierlich entfernt wurde. Anschließend wurde das Lösungsmittel bei vermindertem Druck entfernt, wobei als Rückstand das Ethylenglykolacetal des 2-Chlor-5-nitrobenzaldehyds in nahezu quantitativer Ausbeute anfiel. Smp. einer gereinigten Probe: 88-90°C.

b) 115 g (0,5 mol) des Ethylenglykolacetals aus a) wurden in 1 l Tetrahydrofuran in Anwesenheit von 20 g Raney-Nickel unter einem Wasserstoffdruck von 1,05 bar bei 50°C hydriert. Aus der Reaktionslösung erhielt man nach üblicher Aufarbeitung das Ethylenglykolacetal des 5-Amino-2-chlorbenzaldehyds in fast quantitativer Ausbeute als Öl.

c) Eine Lösung aus 99,8 g (0,5 mol) des 5-Amino-2-chlorbenzaldehydacetals aus b), 76,1 g (0,5 mol) 3,4,5,6-Tetrahydrophthalsäureanhydrid und 0,5 l Eisessig wurde 5 Stunden bei Siedetemperatur gerührt. Das so erhaltene Reaktionsgemisch wurde auf 25°C gekühlt und mit 0,5 l Wasser versetzt, wobei der 2-Chlor-5-(3,4,5,6-tetrahydrophthalimido)benzaldehyd ausfiel. Ausbeute: 83 %; Smp. 140-141°C.

Analog diesem Beispiel wurden die in den nachfolgenden Tabellen 1, 2 und 3 aufgeführten Versuche durchgeführt.

Tabelle 1: Umsetzung der Nitrobenzaldehyde III mit Alkandiolen IV zu den entsprechenden Nitrophenylacetalen V in Toluol in Gegenwart von p-Toluolsulfonsäure (Kat-H)

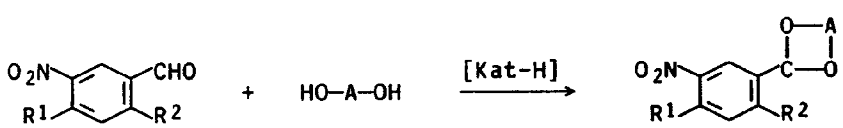


III IV V

| Bsp. Nr. | $R^1$ | $R^2$ | A | Mol III | Mol IV | mMol Kat-H | ml Toluol | T [°C] | Ausbeute V [%] |
|---|---|---|---|---|---|---|---|---|---|
| 2a | F | Cl | $-(CH_2)_3-$ | 0,10 | 0,11 | 0,10 | 250 | 85 | 92 |
| 3a | F | F | $-(CH_2)_3-$ | 0,05 | 0,055 | 0,05 | 200 | 85 | 90 |
| 4a | H | F | $-(CH_2)_2-$ | 0,05 | 0,055 | 0,05 | 200 | 85 | 98 |

Tabelle 2: Reduktion der Nitrophenylacetale V mit Wasserstoff zu den entsprechenden Aminophenylacetalen II in Tetrahydrofuran (THF) mit Wasserstoff in Gegenwart von Raney-Nickel (RaNi)

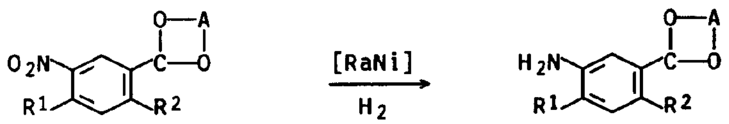


V II

| Bsp. Nr. | Verbindung V | Mol V | Mol $H_2$ | Druck $H_2$ [bar] | RaNi [Gew.% zu V] | THF [ml] | T [°C] | Ausbeute II [%] |
|---|---|---|---|---|---|---|---|---|
| 2b | 2a | 0,05 | 0,15 | 1,05 | 23 | 150 | 40 | 78 |
| 3b | 3a | 0,05 | 0,15 | 1,05 | 24 | 150 | 40 | 98 |
| 4b | 4a | 0,05 | 0,15 | 1,05 | 28 | 150 | 45 | 87 |

Tabelle 3: Kondensation der Aminoacetale II mit 3,4,5,6-Tetrahydrophthalsäureanhydrid (THPA) in Eisessig (HAc) zu den entsprechenden 3-[3,4,5,6-Tetrahydrophthalimido]-benzaldehyden I

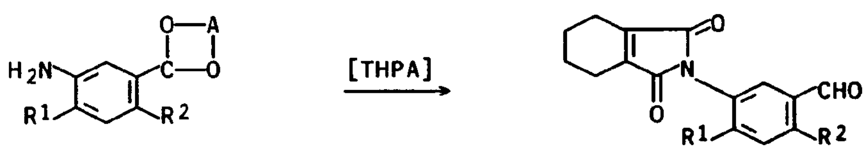


II I

| Bsp. Nr. | Verbindung II | Mol II | Mol THPA | ml HAc | T [°C] | Ausbeute I [%] |
|---|---|---|---|---|---|---|
| 2c | 2b | 0,02 | 0,02 | 100 | 70 | 16 |
| 3c | 3b | 0,01 | 0,01 | 50 | 70 | 69 |
| 4c | 4b | 0,01 | 0,01 | 50 | 70 | 73 |

## Patentansprüche

1. Verfahren zur Herstellung von 3-(3,4,5,6-Tetrahydrophthalimido)-benzaldehyden des Typs I

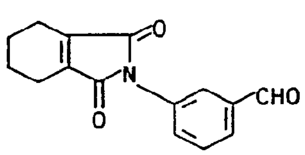

I

oder entsprechenden am Phenylring substituierten Derivaten, wobei als Substituenten Halogen, die Hydroxylgruppe, Alkylgruppen, Alkoxygruppen, Dialkylaminogruppen und Alkoxycarbonylgruppen in Frage kommen und die Alkylreste in diesen Gruppen bis zu 12 C-Atome enthalten und ihrerseits Halogenatome, Hydroxyl- und

$C_1$-$C_4$-Alkoxygruppen tragen können, dadurch gekennzeichnet, daß man

a) die Formylgruppe eines 3-Nitrobenzaldehyds III

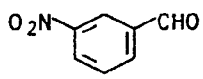

III

oder eines entsprechend am Phenylkern - wie oben genannt - substituierten Derivats in einem inerten organischen Lösungsmittel in Gegenwart eines sauren Katalysators mit einem Alkohol IV

HO-A-OH IV

worin A eine 1,2-Ethylen- oder 1,3-Propylenkette-bedeutet, wobei diese Ketten ihrerseits bis zu drei $C_1$-$C_3$-Alkylgruppen tragen können, zum entsprechenden cyclischen Acetal V

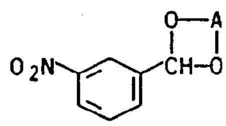

V

kondensiert,

b) das cyclische Acetal V mit Wasserstoff katalytisch zur entsprechenden Aminoverbindung II

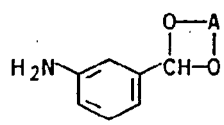

II

reduziert und

c) dieses Aminophenylacetal II in einem sauren Reaktionsmedium mit 3,4,5,6-Tetrahydrophthalsäureanhydrid kondensiert und gleichzeitig die Acetalgruppe in die Aldehydgruppe zurückspaltet.

2. Verfahren nach Anspruch 1, dadurch gekennzeichnet, daß man bei der Acetalisierung des Nitroformylbenzols III als Katalysator p-Toluolsulfonsäure verwendet.

3. Verfahren nach Anspruch 1, dadurch gekennzeichnet, daß man die Kondensation des Aminophenylacetals II mit Tetrahydrophthalsäureanhydrid in Gegenwart einer niederen Carbonsäure durchführt.

4. Neue 3-(3,4,5,6-Tetrahydrophthalimido)-benzaldehyde der allgemeinen Formel Ia

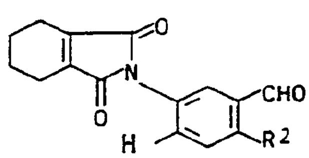

Ia,

in der $R^2$ für Halogen steht.

5. Neue Acetale von 3-Aminobenzaldehyden der allgemeinen Formel IIa

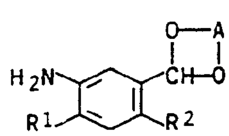

IIa,

in der $R^1$ Wasserstoff oder Halogen, $R^2$ Halogen und A eine 1,2-Ethylen- oder 1,3-Propylenbrücke, die beide ihrerseits bis zu drei $C_1$-$C_3$-Alkylgruppen tragen können, bedeuten.

## Claims

1. A process for the preparation of a 3-(3,4,5,6-tetrahydrophthalimido)-benzaldehyde of the type I

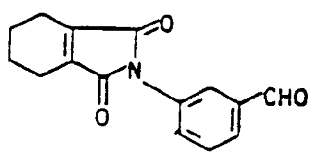

I

or of a corresponding derivative substituted in the phenyl ring, suitable substituents being halogen, hydroxyl, alkyl, alkoxy, dialkylamino or alkoxycarbonyl and the alkyl containing up to 12 carbon atoms and in turn being able to carry halogen atoms and hydroxyl or $C_1$-$C_3$-alkoxy groups, wherein

a) the formyl group of a 3-nitrobenzaldehyde III

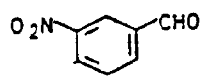

III

or of a corresponding derivative substituted in the phenyl nucleus as indicated above, is condensed with an alcohol IV

HO-A-OH IV

where A is a 1,2-ethylene or 1,3-propylene chain and this chain may in turn carry up to three $C_1$-$C_3$-alkyl groups, in an inert organic solvent in the presence of an acidic catalyst to give the corresponding cyclic acetal V

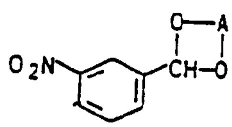

V

b) the cyclic acetal V is reduced catalytically with hydrogen to give the corresponding amino compound II

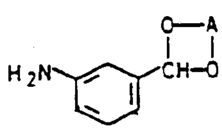

II

and

c) this aminophenyl acetal II is condensed with 3,4,5,6-tetrahydrophthalic anhydride in an acidic reaction medium and at the same time the acetal group is cleaved to give the aldehyde group again.

2. A process as claimed in claim 1, wherein p-toluenesulfonic acid is used as the catalyst in the acetalization of the nitroformylbenzene III.

3. A process as claimed in claim 1, wherein the condensation of the aminophenylacetal II with tetrahydrophthalic anhydride is carried out in the presence of a lower carboxylic acid.

4. A novel 3-(3,4,5,6-tetrahydrophthalimido)-benzaldehyde of the formula Ia

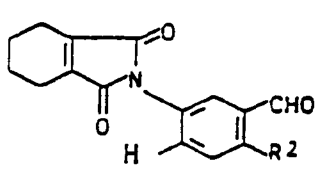

Ia

where $R^2$ is halogen.

5. A novel acetal of a 3-aminobenzaldehyde of the formula IIa

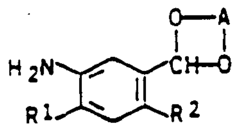

IIa

where $R^1$ is hydrogen or halogen, $R^2$ is halogen and A is al1,2-ethylene or 1,3-propylene bridge both of which may in turn carry up to three $C_1$-$C_3$-alkyl groups.

**Revendications**

1. Procédé de préparation de 3-(3,4,5,6-tétrahydrophtalimido)-benzaldéhydes du type I

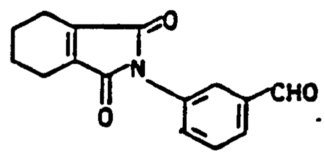

I

ou des dérivés correspondants substitués sur le noyau phényle, les substituants en question étant des halogènes, le groupe hydroxy, des groupes alkyle, des groupes alcoxy, des groupes dialkylamino et des groupes alcoxycarbonyle et les parties alkyle de ces groupes contenant jusqu'à 12 atomes de carbone et pouvant elles-mêmes porter des atomes d'halogènes, des groupes hydroxy et alcoxy en $C_1$-$C_4$, caractérisé en ce que :

a) on condense le groupe formyle d'un 3-nitrobenzaldéhyde III

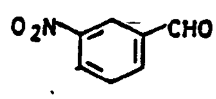

III

ou d'un dérivé correspondant substitué - comme indiqué ci-dessus - sur le noyau phényle, dans un solvant organique inerte, en présence d'un catalyseur acide, avec un alcool IV

HO-A-OH IV

dans laquelle A représente une chaîne 1,2-éthylène ou 1,3-propylène, ces chaînes pouvant elles-mêmes porter jusqu'à trois groupes alkyle en $C_1$-$C_3$, ce qui donne l'acétal cyclique correspondant V

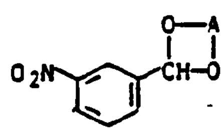

, V

b) on réduit l'acétal cyclique V par l'hydrogène en présence d'un catalyseur en le dérivé aminé correspondant II

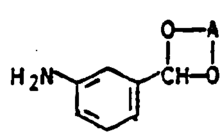

II

et

c) on condense cet aminophénylacétal II dans un milieu de réaction acide avec l'anhydride 3,4,5,6-tétrahydrophtalique et simultanément on reconvertit le groupe acétal en le groupe aldéhyde.

2. Procédé selon la revendication 1, caractérisé en ce que, pour l'acétalisation du nitroformylbenzène III, on utilise en tant que catalyseur l'acide p-toluène sulfonique.

3. Procédé selon la revendication 1, caractérisé en ce que la condensation de l'aminophénylacétal II avec l'anhydride tétrahydrophtalique est effectuée en présence d'un acide carboxylique inférieur.

4. Nouveaux 3-(3,4,5,6-tétrahydrophtalimido)-benzaldéhydes de formule générale Ia

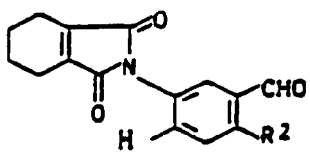

Ia,

dans laquelle $R^2$ représente un halogène.

5. Nouveaux acétals de 3-aminobenzaldéhydes de formule générale IIa

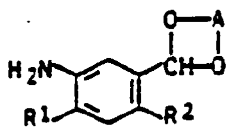

IIa,

dans laquelle $R^1$ représente l'hydrogène ou un halogène, $R^2$ représente un halogène et A un pont 1,2-éthylène ou 1,3-propylène, ces deux ponts pouvant eux-mêmes porter jusqu'à trois groupes alkyle en $C_1$-$C_3$.